# EUROPEAN PATENT APPLICATION

(11) **EP 3 213 722 A1**
(43) Date of publication of application: **06.09.2017**
(21) Application number: 15855512.8
(22) Date of filing: 12.05.2015
(51) Int. Cl.: A61F 2/95, A61F 2/962

(54) **CATHETER FOR STENT OPERATION**

(30) Priority: 29.10.2014 KR 20140147937
(71) Applicant: M.I.Tech Co., Ltd., Pyeongtaek-si, Gyeonggi-do 451-864 (KR)
(72) Inventor: HAN, Jong Hyeon, Seoul 137-762 (KR); PARK, Hun Kuk, Pyeongtaek-si Gyeonggi-do 450-731 (KR); JANG, Bong Seok, Osan-si Gyeonggi-do 447-719 (KR); YUN, Ho, Asan-si Chungcheongnam-do 336-873 (KR)
(74) Representative: Misselhorn, Hein-Martin
(86) International application number: PCT/KR2015/004717
(87) International publication number: WO 2016/068425

(57) **Abstract**

The present invention relates to a catheter for a stent operation. The catheter for a stent operation according to the present invention comprises: a solid bar of which one end is coupled to the side of a handle; an outer tube into which the solid bar is inserted, having an insertion hole through which a guide wire can be inserted from the outside to the inside, and of which one end is coupled to the side of the body; a guide tip coupled to the side of the solid bar, and guiding the guide wire by having a through-hole through which the guide wire passes; a connection tube for connecting and coupling the guide tip to the solid bar, wherein the solid bar has an accommodation groove, for preparing a space capable of accommodating the guide wire, formed in a predetermined section of a region to be connected to the connection tube, such that the guide wire can be accommodated inside of the outer tube. According to the present invention, the length of the guide wire is short but the structure is simple due to the rod-shaped solid bar, and thus there are effects of improving productivity by having a simple assembly and reducing the defect rate, and preventing operation defects.

## Description

### [Technical Field]

The present invention relates to a catheter for inserting a stent into a lumen of a human body.

### [Background Art]

A stent is used to operate when a lumen becomes narrow due to the growth of lesion tissue and thus food is not smoothly moved. The stent may be made of a superelastic shape memory alloy and can keep the passageway of the narrowed lumen open due to its elasticity.

A catheter is used to insert such a stent into a lumen.

The catheter has a structure that is able to place a stent mounted therein with a minimized radius into the region of a lesion and then expose the stent. The exposed stent inflates due to its restoring force and thus keeps the passageway of the region of the lesion open. In order to place the stent into the region of a lesion during an operation, a guide wire is required.

In the prior art, the length of the guide wire should be two times longer than the catheter by necessity. Such a long guide wire has a problem that it hinders a patient from freely moving. Therefore, Korean Patent Publication No. 10-2012-0079431 (titled "Catheter Structure for Stent Surgical Operation In Using Short Guide Wire Stent", hereinafter, referred to as a "prior art") discloses a technology of reducing the length of a guide wire.

In the prior art, a guide tube is coupled to the inside of an inner tube by being inserted thereinto. Herein, the guide tube functions to guide the guide wire to be bent. According to such a prior art, since a short guide wire can be used, there is an effect accompanied therewith (minimized inconvenience of a patient, easiness of an operation, or the like).

However, since the guide tube should be inserted into the inside of the inner tube having a very narrow passageway to be coupled thereto, the structure is complicated and productivity is reduced, and a defect rate is high. In addition, when the inner tube and an outer tube move relative to each other according to a relative movement of a grip body and a handle, there may be a defect in manipulation due to the bent inner tube.

### [Detailed Description of the Present Disclosure]

### [Technical Objects]

An object of the present invention is to provide a technology which can have the advantage of a short guide wire as it is and also is provided with a rod-shaped structure rather than a tube inside an outer tube.

### [Technical Solving Means]

According to a first aspect of the present invention to achieve the above-described object, a catheter for a stent operation includes: a solid bar which has one end coupled to the side of a handle; an outer tube into which the solid bar is inserted, the outer tube having an insertion hole through which a guide wire is inserted from the outside to the inside, and having one end coupled to the side of a body; a guide tip which is coupled to the side of the solid bar and guides the guide wire by having a through-hole through which the guide wire passes; and a connection tube which connects and couples the guide tip to the solid bar, wherein the guide wire is inserted from the outside of the outer tube to the inside through the insertion hole, and then passes through the inside of the connection tube and escapes through the through-hole of the guide tip, and wherein the solid bar has an accommodation groove formed in a predetermined section of a region to be connected to the side of the connection tube, for providing a space for accommodating the guide wire, such that the guide wire is accommodated inside the outer tube.

The catheter may further include a guide member which couples the solid bar and the connection tube to each other, and guides the guide wire inserted through the insertion hole of the outer tube to pass through the inside of the connection tube and to escape through the through-hole of the guide tip. The guide member may have a guide hole for guiding the guide wire, and the guide hole may have a first guide region which is disposed on the same straight line as the through-hole of the guide tip, for guiding the guide wire to the through-hole, and a second guide region which extends from the first guide region and bends toward the insertion hole in order to bend the guide wire inserted through the insertion hole of the outer tube and to guide the guide wire to the first guide region.

The guide member may be integrally formed with the solid bar.

The solid bar may include a guide end having a guide hole disposed at a region coupled to the connection tube on the same straight line as the through-hole of the guide tip, for guiding the guide wire to the through-hole.

The catheter may further include a guide tube which guides the guide wire inserted through the insertion hole of the outer tube to be inserted into the inside of the connection tube through the guide hole, and the guide tube may be coupled to the outer tube.

According to a second aspect of the present invention to achieve the above-described object, a catheter for a stent operation includes: a solid bar which has one end coupled to the side of a handle; an outer tube into which the solid bar is inserted, the outer tube having an insertion hole through which a guide wire is inserted from the outside to the inside, and having one end coupled to the side of a body; a guide tip which is coupled to the side of the solid bar and guides the guide wire by having a through-hole through which the guide wire passes; a connection tube which connects and couples the guide tip to the solid bar; and a guide tube which guides the guide wire inserted through the insertion hole of the outer tube to be inserted into the inside of the connection tube, and wherein the guide tube is coupled to the outer tube by being inserted into the outer tube. The solid bar may have an accommodation groove formed in a predetermined section of a region to be connected to the side of the connection tube, for providing a space for accommodating the guide wire, such that the guide wire is accommodated inside the outer tube.

The solid bar may have a guide end having a guide hole formed therein, for guiding the guide wire to the through-hole.

### [Effect of the Invention]

According to the present invention, the following effects can be achieved by having a short guide wire and also having a rod-shaped solid bar:
First, since the structure is simple, an assembly is simple, and a defect rate is reduced and thus productivity is improved.
Second, when the solid bar and the outer tube move in opposite directions, there is an effect that a manipulation defect which may be caused by the bent solid bar can be minimized.

### [Brief Description of Drawings]

FIG. 1 is a schematic perspective view of a catheter according to a first embodiment of the present invention;
FIG. 2 is an exploded cross section view of main portions of the catheter of FIG. 1;
FIG. 3 is an assembled cross section view of the main portions of the catheter of FIG. 1;
FIGs. 4 and 5 are reference views to illustrate a using state of the catheter of FIG. 1;
FIG. 6 is a cross section view of a solid bar of a catheter applying the first embodiment;
FIG. 7 is an exploded perspective view of main portions of a catheter according to a second embodiment of the present invention; and
FIG. 8 is an assembled cross section view of the main portions of the catheter of FIG. 7.

### [Best Mode for Embodying the Invention]

Hereinafter, preferred embodiments according to the present invention as described above will be described with reference to the accompanying drawings, but a redundant description will be omitted or descriptions will be condensed for a brief explanation.

### <First Embodiment>

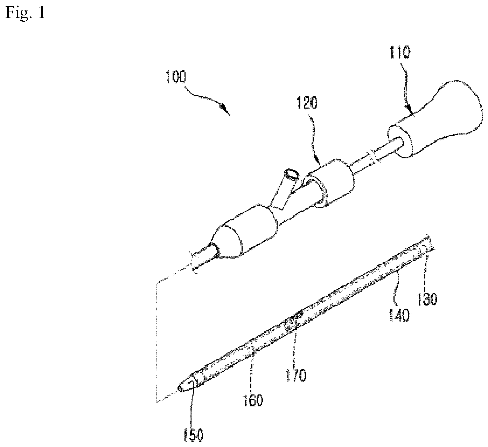

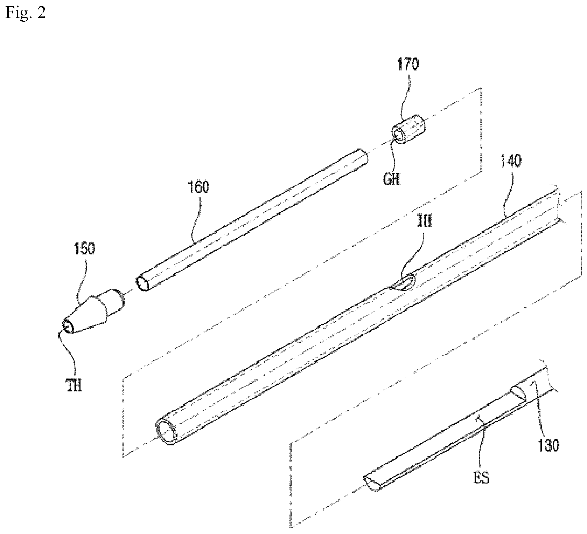

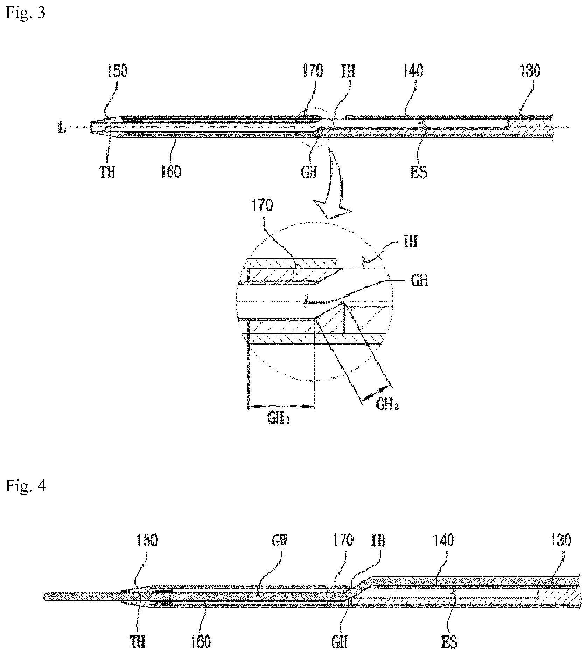

FIG. 1 is a schematic perspective view of a catheter 100 for a stent operation (hereinafter, referred to as a "catheter") according to a first embodiment of the present invention. FIG. 2 is an exploded cross section view of main portions of the catheter 100 of FIG. 1, and FIG. 3 is an assembled cross section view of the main portions of the catheter 100 of FIG. 1.

As shown in FIG. 1, the catheter 100 according to the first embodiment of the present invention includes a solid bar 130, an outer tube 140, a guide tip 150, a connection tube 160, and a guide member 170.

The solid bar 130 has one end coupled to the side of a handle 110 and the other end coupled to the side of the connection tube 160. Unlike a tubular shape, the solid bar 130 has a rigid rod shape which is not hollow and is filled. Accordingly, a guide wire cannot be inserted into the inside of the solid bar 130. Accordingly, the solid bar 130 has an accommodation groove ES formed thereon for providing a space to have the guide wire accommodated inside the outer tube 140 when the outer tube 140 is withdrawn relative to the solid bar 130. Herein, the accommodation groove ES is formed over a predetermined section of a region to be connected to the side of the connection tube 160. The accommodation groove ES may be formed by simply cutting a predetermined section of the region of the other side of the solid bar 130.

The solid bar 130 is inserted into the inside of the outer tube 140. In addition, the outer tube 140 has an insertion hole IH to allow the guide wire to be inserted from the outside to the inside.

In addition, the outer tube 140 has one end coupled to the side of a body 120. Accordingly, when a user moves the handle 110 and the body 120 relative to each other in opposite directions, the solid bar 130 and the outer tube 140 move relative to each other in opposite directions.

The guide tip 150 is coupled to the side of the solid bar 130 with the connection tube 160 disposed therebetween, and guides the guide wire by having a through- hole TH through which the guide wire passes.

The connection tube 160 connects and couples the guide tip 150 to the solid bar 130. That is, one side of the connection tube 160 is coupled to the guide member 170 coupled to the solid bar 130, and the other side of the connection tube 160 is coupled to the guide tip 150.

The guide member 170 has a guide hole GH to guide the guide wire. Such a guide hole GH may be divided into a first guide region GH₁ and a second guide region GH₂.

The first guide region GH₁ is disposed on the same straight line L as the through-hole TH of the guide tip 150, thereby guiding the guide wire to the through-hole TH.

The second guide region GH₂ bends the guide wire inserted through the insertion hole IH of the outer tube 140 and guides the guide wire to the first guide region GH₁. To achieve this, one side of the second guide region GH₂ faces the insertion hole IH and the other side of the second guide region GH₂ is connected to the first guide region GH₁. That is, the second guide region GH₂ extends from the first guide region GH₁ and bends toward the insertion hole TH.

As shown in FIG. 4, in the catheter 100 having the above-described structure, the guide wire GW which is inserted from the outside of the outer tube 140 to the inside through the insertion hole IH passes through the guide hole GH and the inside of the connection tube 160 and then escapes through the through-hole TH of the guide tip 150.

Next, a method for performing a stent operation using the above-described catheter 100 will be described.

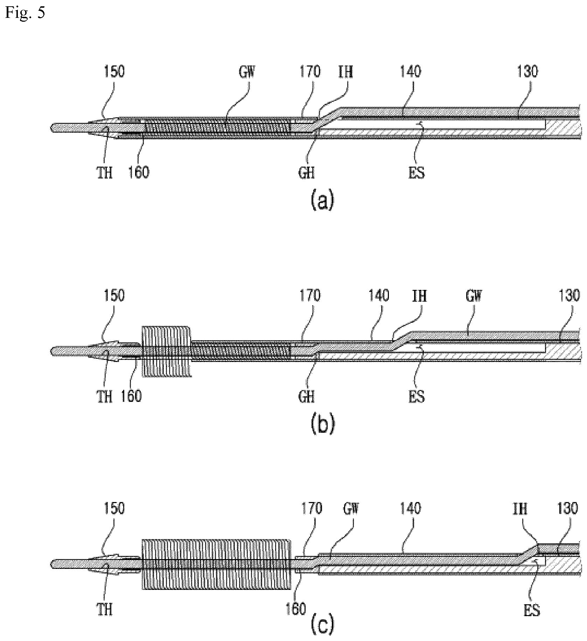

First, a stent ST is placed on a region of a lesion using the guide wire GW as shown in view (a) of FIG. 5.

Next, the outer tube 140 is withdrawn from the guide tip 150 by pulling the body 120 toward the handle 110 as shown in views (b) and (c) of FIG. 5. In this case, since the guide wire GW and the solid bar 130 remain still, the guide wire GW accommodated in the outer tube 140 is accommodated in the accommodation groove ES when the outer tube 140 is withdrawn. More specifically, when the outer tube 140 is withdrawn, a predetermined section of the guide wire GW relatively entering the inside of the outer tube is accommodated in the accommodation groove ES.

Herein, when the outer tube 140 is withdrawn, the solid bar 130 of the rod shape is not easily crumpled or bent by a friction with the outer tube 140. Accordingly, the outer tube 140 can be stably moved in the opposite direction relative to the solid bar 130. When the outer tube 140 is sufficiently withdrawn as shown in view (c) of FIG. 5, the stent ST compressed between the outer tube 140 and the connection tube 160 is completely released by the outer tube 140 and applies an expansive force to the region of the lesion.

### <Example of Application>

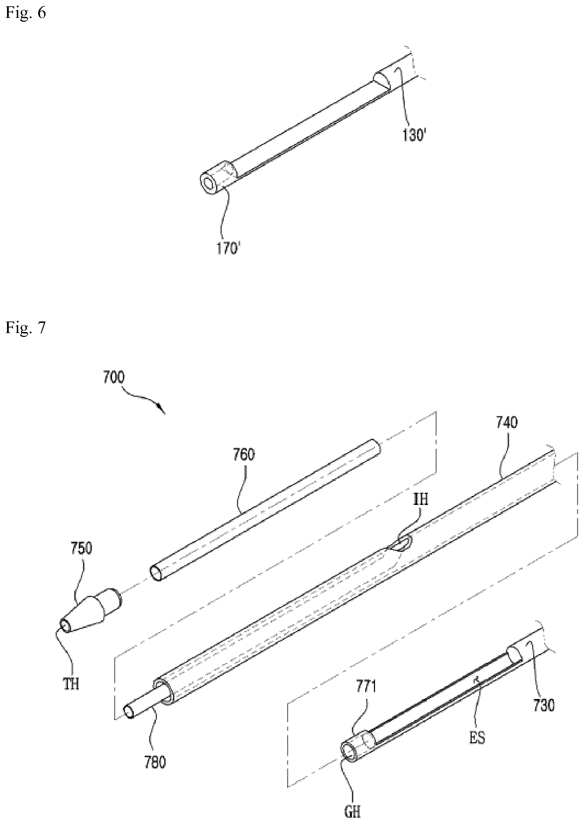

The catheter 100 according to the first embodiment may be applied to have a form in which the guide member 170 is integrally formed with the solid bar 130. Accordingly, according to such an application example, a guide member 170' may be integrally formed with a solid bar 130' as shown in FIG. 6. Of course, the other configurations except for this difference are the same as in the first embodiment.

### <Second Embodiment>

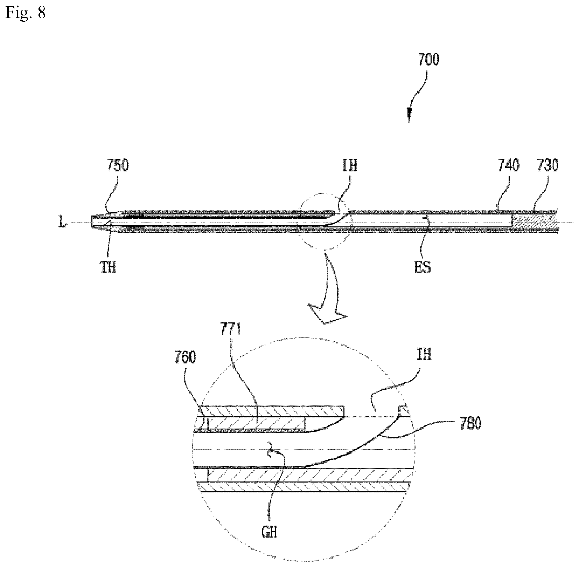

FIG. 7 is an exploded cross section view of main portions of a catheter 700 according to a second embodiment of the present invention, and FIG. 8 is an assembled cross section view of the main portions of the catheter 700 according to the second embodiment of the present invention.

The catheter 700 according to the second embodiment of the present invention includes a solid bar 730, an outer tube 740, a guide tip 750, a connection tube 760, and a guide tube 780.

In the present embodiment, the outer tube 740, the guide tip 750, and the connection tube 760 are the same as the outer tube 140, the guide tip 150, and the connection tube 160 in the first embodiment, and thus a detailed description thereof is omitted.

The solid bar 730 has a guide end 771 formed at the other end thereof which is a region coupled to the connection tube 760. In addition, the guide end 771 has a guide hole GH which is disposed on the same straight line L as the through-hole TH of the guide tip 750 to guide the guide wire to the through-hole TH. The solid bar 730 according to the present embodiment also has an accommodation groove ES formed thereon to accommodate the guide wire.

The guide tube 780 guides the guide wire inserted through the insertion hole IH of the outer tube 740 to be inserted into the inside of the connection tube 760 through the guide hole GH. One side of the guide tube 780 is coupled to the outer tube 740 on the region of the insertion hole IH, and the other side of the guide tube 780 extends toward the guide tip 750. Since such a guide tube 780 smoothly guides the bending of the guide wire when the outer tube 740 moves, a manipulation defect of the guide wire in the insertion hole IS can be prevented.

As described above, the present invention has been described in detail by embodiments referring to the accompanying drawings. However, the above-described embodiments have been just described based on preferred examples of the present invention. Accordingly, the present invention should not be understood as being limited to the above-described embodiments and the scope of the present invention should be understood as being defined by the appended claims and equivalents thereto.

### (Explanation of Signs)

100, 700: catheter for a stent operation
110: handle 120: body
130: solid bar
ES: accommodation groove
140: outer tube
IS: insertion hole
150: guide tip
TH: through-hole
160: connection tube
170: guide member
GH: guide hole
GH₁: first guide region GH₂: second guide region 780: guide tube
GW: guide wire

## Claims

1. A catheter for a stent operation, comprising:
a solid bar which has one end coupled to the side of a handle;
an outer tube into which the solid bar is inserted, the outer tube having an insertion hole through which a guide wire is inserted from the outside to the inside, and having one end coupled to the side of a body;
a guide tip which is coupled to the side of the solid bar and guides the guide wire by having a through-hole through which the guide wire passes; and
a connection tube which connects and couples the guide tip to the solid bar,
wherein the guide wire is inserted from the outside of the outer tube to the inside through the insertion hole, and then passes through the inside of the connection tube and escapes through the through-hole of the guide tip, and
wherein the solid bar has an accommodation groove formed in a predetermined section of a region to be connected to the side of the connection tube, for providing a space for accommodating the guide wire, such that the guide wire is accommodated inside the outer tube.

2. The catheter of claim 1, further comprising a guide member which couples the solid bar and the connection tube to each other, and guides the guide wire inserted through the insertion hole of the outer tube to pass through the inside of the connection tube and to escape through the through-hole of the guide tip,
wherein the guide member has a guide hole for guiding the guide wire, and
wherein the guide hole has a first guide region which is disposed on the same straight line as the through-hole of the guide tip, for guiding the guide wire to the through-hole, and a second guide region which extends from the first guide region and bends toward the insertion hole in order to bend the guide wire inserted through the insertion hole of the outer tube and to guide the guide wire to the first guide region.

3. The catheter of claim 2, wherein the guide member is integrally formed with the solid bar.

4. The catheter of claim 1, wherein the solid bar comprises a guide end having a guide hole disposed at a region coupled to the connection tube on the same straight line as the through-hole of the guide tip, for guiding the guide wire to the through-hole.

5. The catheter of claim 4, further comprising a guide tube which guides the guide wire inserted through the insertion hole of the outer tube to be inserted into the inside of the connection tube through the guide hole, and
wherein the guide tube is coupled to the outer tube.

6. A catheter for a stent operation, comprising:
a solid bar which has one end coupled to the side of a handle;
an outer tube into which the solid bar is inserted, the outer tube having an insertion hole through which a guide wire is inserted from the outside to the inside, and having one end coupled to the side of a body;
a guide tip which is coupled to the side of the solid bar and guides the guide wire by having a through-hole through which the guide wire passes;
a connection tube which connects and couples the guide tip to the solid bar; and
a guide tube which guides the guide wire inserted through the insertion hole of the outer tube to be inserted into the inside of the connection tube, and
wherein the guide tube is coupled to the outer tube by being inserted into the outer tube.

7. The catheter of claim 6, wherein the solid bar has an accommodation groove formed in a predetermined section of a region to be connected to the side of the connection tube, for providing a space for accommodating the guide wire, such that the guide wire is accommodated inside the outer tube.

8. The catheter of claim 7, wherein the solid bar has a guide end having a guide hole formed therein, for guiding the guide wire to the through-hole.
